# EUROPEAN PATENT SPECIFICATION

(11) **EP 1 728 434 B1**
(45) Date of publication and mention of the grant of the patent: **24.12.2008**
(21) Application number: 05011690.4
(22) Date of filing: 31.05.2005
(51) Int. Cl.: A23G 1/00

(54) **Process for the manufacture of a polyphenol-enriched composition from cocoa shell extraction**
Verfahren zur Herstellung von Polyphenol angereicherten Zusammensetzungen aus Kakaoschale
Procédé de production d'une composition enrichie en polyphenol extrait d'enveloppe de cacao

(43) Date of publication of application: 06.12.2006
(73) Proprietor: Kraft Foods R & D, Inc., Northfield, IL 60093 (US)
(72) Inventor: Bradbury, Allan, 85244 Röhrmoos (DE); Kopp, Gabriele, 81825 München (DE)
(74) Representative: HOFFMANN EITLE

(56) References cited:
- EP-A- 1 346 640
- US-A1- 2003 180 406
- US-A1- 2004 096 566

## Description

### FIELD OF THE INVENTION

The present invention relates to a process for the extraction of cocoa shells to provide a theobromine-enriched fraction or composition and a polyphenol-enriched fraction or composition.

### BACKGROUND OF THE INVENTION

Naturally-occurring polyphenols derived from plants or plant materials (e.g, tea, cocoa beans, and the like) are known to have antioxidant properties as well as providing other potential health benefits. Thus, considerable research has been carried out in recent years with regard to methods for obtaining such polyphenols, including cocoa polyphenols, as well as methods for using them.

U.S. Patent 5,554,645 (September 10, 1996) provides a method for extraction of polyphenols from dehulled, defatted, freeze-dried cocoa bean powder using a 70:30 acetone:water solvent. The extract was then purified using gel permeation chromatography or high performance liquid chromatography to obtain polyphenols consisting essentially of oligomers 3 through 12.

International Patent Publication WO 00/45769 (August 10, 2000) and corresponding U.S. Patent 6,576,275 (June 10, 2003) provide a method for extraction of polyphenols from cocoa and other plant materials to provide a reduced-purine-containing polyphenol material. Starting cocoa materials including, for example, fresh cocoa beans, defatted cocoa solids, cocoa powder, low-fat cocoa powder, cocoa shells, cocoa waste materials, or other cocoa-containing raw materials are preferably ground to a particle size of less than 250:m. The ground cocoa material is then extracted with a hydroxylic solvent (e.g., water, lower alcohols, and mixtures thereof). The resulting extract is then applied to an adsorption material having a high polyphenol/purine adsorption ratio of at least about 5/1. Suitable absorbents include polyvinylpolypyrrolidone and chitosan, as well as derivatives, modifications, and blends thereof. The polyphenols are then removed from the adsorption material using a desorption solvent such as water, a water-miscible alcohol, mixtures of water and a water-miscible alcohol, and mixtures of water and a water-miscible ketone. The polyphenol-containing material can then be concentrated by evaporation of the desorption solvent. Although this method reduces the relative amounts of purines (i.e., theobromine and caffeine), significant amounts of purines remain in the collected polyphenol-containing materials.

U.S. Patent 6,159,451 (December 12, 2000) provides a method of producing a fraction of cacao bean husk having activity against glucosyltranferase in the prevention of tooth decay. In this method, 4 to 10 parts of a 50 percent acetone aqueous solution was added to 1 part dried cacoa bean husk, stirred under reflux at 40 to 80°C for 4 to 6 hours, to form an extract. This procedure was repeated twice. The extract was then concentrated and dried using vacuum techniques. The resulting extract was then added to a styrene-based adsorption resin, washed with 1 to 2 parts of 20 percent ethanol, followed by the addition of 1 to 2 parts of 50 percent ethanol. The fractions from the 50 percent ethanol elution were collected and concentrated under reduced pressure at 40 to 50°C to provide the desired fraction having activity against glucosyltranferase.

International Patent Publication WO 00/62631 (October 26, 2000) provides a method to produce a cacao extract containing dietary fiber (especially insoluble dietary fiber) which is reported to be useful in treating diabetes. Heat-treated cacao husks are extracted with ethanol and then centrifuged and then fractionated into a supematant and a residue. The residue faction is reportedly useful in the treatment of diabetes. The supernatant is reported to contain polyphenols and can be used in beverages.

European Patent Application EP 1304047 (published April 23, 2003) provides a method for obtaining an extract of cacao bean or cacoa bean husks which reportedly inhibits the suppression of gap junctional intercellular communication and DNA synthesis of cancer cells. After removing cocoa butter, the cacao bean husks were extracted with 50 percent acetone for 5 hours at 60°C under reflux. The extract was centrifuged and the supematant was collected, filtered, concentrated, and freeze dried. The resulting cacao bean husk fraction was adsorbed on a polystyrene hydrophobic adsorption resin and fractionated with water/acetone or water/methanol mixtures to obtain the active material.

European Patent Application EP 1346640 (published September 24, 2003) provides a method for producing a low fat cocoa extract having a high level of cocoa flavors and/or cocoa flavor precursors and a high level of antioxidants (e.g., polyphenols). This method involves placing cocoa seeds in an acetic acid solution, heating the mixture, removing the seeds, and concentrating the dissolved solids from the solution.

U.S. Patent 6,627,232 (September 30, 2003) provides a method for selectively extracting tetramers, pentamers, and higher molecular weight cocoa procyanidin oligomers from partially defatted or fully defatted cocoa solids prepared from cocoa beans that have not been roasted. In one embodiment, this method includes the steps of (a) extracting the cocoa solids with ethyl acetate; (b) recovering the extracted cocoa solids; (c) extracting the recovered, extracted cocoa solids with a solvent selected from the group consisting of acetone, ethanol and mixtures thereof with up to 50% water by volume and; (d) separating the cocoa solids from the cocoa extract of step (c) to obtain the procyanidin extract.

U.S. Patent Publication US 2004/0096566 (May 20, 2004) provides a method for obtaining polyphenols from cocoa beans. The fresh beans (which have not been pre-treated or defatted) are treated to remove the shells and pulp, ground in the presence of solvents, infused with the solvent for a few hours to a few days, filtered and rinsed with the same solvent, and the solvent removed by distillation under vacuum to obtain the desired residue.

Although these methods are generally able to provide polyphenols from various portions of the cocoa bean, it would still be desirable to provide a methods in which a polyphenol-enriched fraction and a theobromine-enriched fraction can be obtained so the more of the components can be used. Moreover, it would be desirable to provide such fractions using what is normally considered a waste product (namely, the cocoa shells). The present invention provides such methods.

### SUMMARY OF THE INVENTION

The present invention relates to a process for the manufacture of a theobromine-enriched composition and a polyphenol-enriched composition from cocoa shells, said process comprising
(1) providing defatted cocoa shells;
(2) extracting the defatted cocoa shells with an aqueous acetone solution containing about 30 to about 80 percent acetone to provide a cocoa shell extract material;
(3) treating the cocoa shell extract material to remove suspended solids to provide a treated cocoa shell extract material;
(4) removing acetone from the treated cocoa shell extract material to provide an aqueous extract material;
(5) concentrating the aqueous extract material to about 1.5 to about 5 percent solids to provide a concentrated extract material;
(6) applying the concentrated extract material to a gel filtration column containing a gel filtration medium suitable for separating theobromine from polyphenols;
(7) rinsing the gel filtration column with water to collect a theobromine-enriched fraction;
(8) eluting the water-rinsed gel filtration column with a low molecular weight polar organic solvent to collect a polyphenol-enriched fraction, wherein the polyphenol-enriched fraction is essentially free of phytosterols and theobromine;
(9) concentrating the theobromine-enriched fraction to obtain the theobromine-enriched composition; and
(10) concentrating the polyphenol-enriched fraction to obtain the polyphenol-enriched composition. Preferred gel filtration media for use in this invention include Sephadex™-type gel filtration media from Amersham Biosciences (a part of GE Healthcare); Sephadex™ LH-20 is especially preferred. The low molecular weight polar organic solvent is preferably methanol. The cocoa shells used in the present invention can be derived from roasted or unroasted cocoa beans and/or from fermented or unfermented cocoa beans.

### BRIEF DESCRIPTION OF THE FIGURES

Figure 1 is a general flow diagram illustrating the present invention.
Figure 2 is a detailed flow diagram illustrating a preferred embodiment of the present invention.

### DETAILED DESCRIPTION

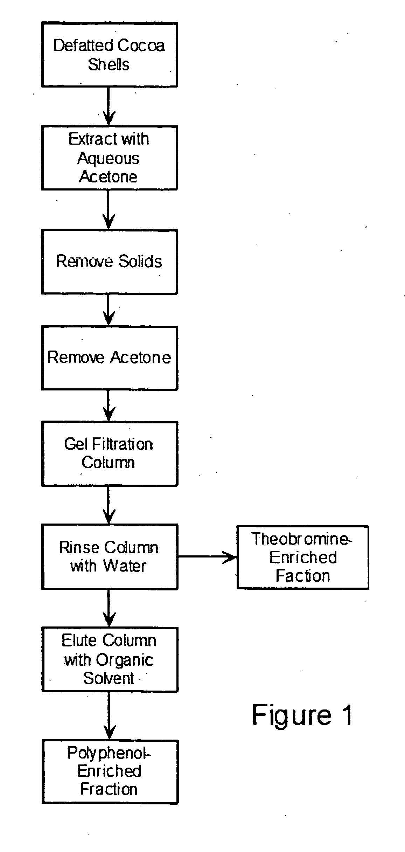

As indicated in Figure 1, defatted cocoa shells are obtained. The cocoa shells may be derived from fermented or unfermented beans, either of which may be unroasted or roasted; the shells used in the extraction process of this invention may be whole, broken, ground, or combinations thereof. Such defatted cocoa shells can be prepared using conventional techniques; for example, the cocoa shells can be defatted by extraction using hexane or other low molecular weight, non-polar or weakly-polar solvent (e.g., pentane, ethyl ether, petroleum ether, and the like); the non-polar solvent hexane is generally preferred. Although not wishing to be limited by theory, it is thought that defatting the cocoa shells assists in removing phytosterols prior to the next extraction step as well as providing for improved separation in the later stages of the process. The defatted cocoa shells are extracted with an aqueous acetone solution. Generally, this aqueous acetone solution contains about 30 to about 80 percent acetone, preferably about 40 to about 60 percent acetone, and more preferably about 50 percent acetone. Suspended solids are then removed from the aqueous acetone extracted material using conventional techniques (e.g., filtration, decantation, centrifugation, and the like). At least a portion of the acetone (generally about 80 percent or more and preferably about 95 to essentially 100 percent) is removed using conventional techniques (e.g., distillation, vacuum distillation, and the like) to obtain an aqueous extract material. The resulting aqueous extract material is then subjected to gel filtration using a gel filtration medium suitable for separating both theobromine and polyphenols. Preferred gel filtration media for use in this invention include Sephadex™-type gel filtration media (generally derived from three-dimensional crosslinked polysaccharide dextran) from Amersham Biosciences (a part of GE Healthcare); Sephadex™ LH-20 is especially preferred. The gel filtration column containing the aqueous extract material is first rinsed with water to remove a theobromine-enriched fraction; the theobromine-enriched fraction is collected. The gel filtration column is then eluted with a low molecular weight polar organic solvent to remove the polyphenol-enriched fraction; the polyphenol-enriched fraction is then collected. Suitable low molecular weight polar organic solvents include, for example, methanol, ethanol, ethyl acetate, acetone, and the like; generally, methanol is preferred.

The polyphenol-enriched fraction is essentially free of phytosterols and theobromine. For purposes of this invention, "essentially free of phytosterols" is intended to include polyphenol-enriched compositions wherein at least about 90 percent, preferably about 95, and most preferably essentially 100 percent (i.e., only a trace remaining), of the phystosterols present in the original cocoa shells have been removed. Likewise, for purposes of this invention, "essentially free of theobromine" is intended to include polyphenol-enriched compositions wherein at least about 90 percent, preferably about 95, and most preferably essentially 100 percent (i.e., only a trace remaining), of the theobromine present in the original cocoa shells have been removed.

As noted above, the cocoa shells used in the present invention can be derived from roasted or unroasted cocoa beans and/or from fermented or unfermented cocoa beans. Generally, higher yields of polyphenols are expected from unfermented shells as compared to fermented shells as well as from roasted shells as compared to unroasted shells.

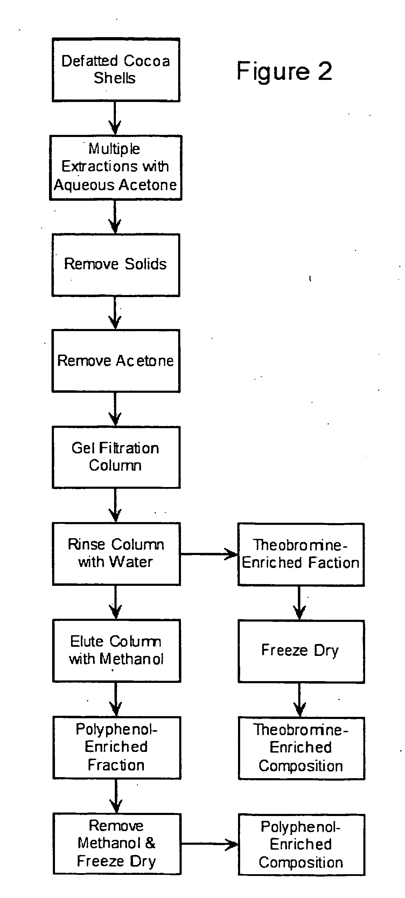

Figure 2 illustrates a preferred embodiment of the present invention. In this embodiment, the defatted cocoa shells are extracted multiple times with an aqueous acetone solution. Generally, this aqueous acetone solution contains 30 to about 80 percent acetone, preferably about 40 to about 60 percent acetone, and more preferably about 50 percent acetone. For purposes of this invention, "multiple times" is intended to mean at least two extractions, preferably about 2 to 5 extractions, and more preferably about 2 to 3 extractions. Preferably, each extraction is carried out with a fresh (i.e., one which has not been previously used for extraction) aqueous acetone solution. The liquid extracts are preferably combined and then treated to remove suspended solids using conventional techniques (e.g., filtration, decantation with or without centrifugation, and the like). At least a portion of the acetone (generally about 80 percent or more and preferably about 95 to essentially 100 percent) is removed using conventional techniques (e.g., distillation, vacuum distillation, and the like) to obtain an aqueous extract material. The resulting aqueous extract material is then subjected to gel filtration using a gel filtration medium suitable for separating both theobromine and polyphenols. Preferred gel filtration media for use in this invention include Sephadex™-type gel filtration media (generally derived from three-dimensional crosslinked polysaccharide dextran) from Amersham Biosciences (a part of GE Healthcare); Sephadex™ LH-20 is especially preferred. The gel filtration column containing the aqueous extract material is first rinsed with water to remove a theobromine-enriched fraction; the theobromine-enriched fraction is collected. The gel filtration column is then eluted with a low molecular weight polar organic solvent, preferably methanol, to remove the polyphenol-enriched fraction; the polyphenol-enriched fraction is then collected.

The collected theobromine-enriched fraction can be used as the theobromine-enriched composition or, if desired, concentrated to obtain the theobromine-enriched composition. Such concentration can be carried out using conventional techniques such as, for example, freeze drying, thermal drying, crystallization, liquid partition techniques using chloroform or methylene chloride, and the like; generally freeze drying is preferred.

The collected polyphenol-enriched fraction can be used as the polyphenol-enriched composition or, if desired, concentrated to obtain the polyphenol-enriched composition. Such concentration can be carried out by first removing at least a portion of the methanol using conventional techniques (e.g., evaporation, vacuum distillation, and the like) followed by removing at least a portion of the water by conventional techniques (e.g., freeze drying, thermal drying, crystallization, liquid partition, and the like) with freeze drying being preferred.

The invention will now be illustrated by specific examples which describe preferred embodiments of the present invention. They are not intended to limit the scope of the invention. Unless otherwise indicated, all ratios and percentages throughout this specification are by weight. All patents and other publications discussed in this specification are hereby incorporated by reference.

**Example 1.** This example illustrates the isolation of a polyphenol-enriched fraction and a theobromine-enriched fraction from cocoa shells. Cocoa shells were obtained from unroasted fermented cocoa beans in the usual way. Roasted or unroasted cocoa shells from fermented or unfermented cocoa beans can be used in the same manner if desired. The cocoa shells (60g) were extracted with hexane (200 ml) for 6 hours in a soxhlet extractor. After drying in vacuum, the defatted shells were slurried with 50 percent aqueous acetone (350 ml) at 70°C for 45 minutes in a flask fitted with a condenser. After centrifuging and decanting the extract, the residue was further extracted with 50% acetone solution (200ml); the extraction/separation procedure was repeated up to 4 times. The extracts were combined and concentrated to about 2X by rotary evaporation. The resulting concentrated aqueous solution was taken to dryness by freeze drying. Polyphenol contents were measured by the Folin-Ciocalteu method (see, e.g., Singleton et al., Am. J. Enol. Vit., 37, 144-158 (1965)). The following results were obtained:

| **Number of Extractions** | **Relative Polyphenol Content (%) of Extract** |
|---|---|
| 1 | 49 |
| 2 | 28 |
| 3 | 9 |
| 4 | 7 |
| 5 | 7 |

The freeze dried concentrated extract obtained from combining all five extractions was added to a Sephadex LH-20 gel permeation column (diameter 4.5 cm, length 30 cm). The column was rinsed with water to yield a theobromine-enriched fraction which was obtained in the dry state by freeze drying. Methanol was then used to elute a polyphenol-enriched fraction which was then dried by evaporation of the methanol and freeze drying to obtain a solid polyphenol-enriched composition or residue.

The following results were obtained:

| **Fraction** | **wt. (g/100g shells)** | **Polyphenols** | | **Theobromine (mg/100g shells)** | **Caffeine (mg/100g shells)** |
|---|---|---|---|---|---|
| | | **(% of fraction)** | **(g/100g shells)** | | |
| 50% acetone | 15.0 | 12.7 | 1.90 | 680 | 65 |
| Water eluate | 12.7 | 5.9 | 0.75 | 665 | 60 |
| MeOH eluate | 1.37 | 56.3 | 0.77 | 2 | 2 |

This example dearly demonstrates that a theobromine-enriched fraction (i.e., the water eluate) and a polyphenol-enriched faction (i.e, the methanol eluate) can be obtained from cocoa shells using the present invention. Moreover, this example also demonstrates that the polyphenol-enriched fraction is essentially free of theobromine (as well as caffeine). After the methanol elution, polyphenols remain on the column (about 1.90 - 0.75 - 0.77 = 0.38 g/100g shells); further recovery of polyphenols could be obtained, if desired, by additional methanol elution of the column and treatment of the eluate as above.

The antioxidant capacity of the polyphenol-enriched fraction methanol was measured using a TEAC (Trolox Equivalent Antioxidant Capacity) method (Re et al., Free Radical Biology & Medicine, 26, 1231-1237 (1999)). An antioxidant capacity of 1.8 µmol Trolox/mg was found. This antioxidant capacity is significantly higher than that typically found for commercial rosemary, grape, and elderberry extract samples (0.83, 0.76 and 0.42 µmol Trolox/mg, respectively).

**Example 2.** This example illustrates a modification of the procedure used in Example 1. Essentially the same procedure was used as in Example 1 except that, subsequent to water rinsing, the column was eluted with 50% aqueous methanol followed by pure methanol. The eluants were dried as described in Example 1.

The following results were obtained:

| **Fraction** | **Yield (g/100g shells)** | **Polyphenols (% of fraction)** |
|---|---|---|
| Water eluate | 12.7 | 5.31 |
| 50% MeOH eluate | 0.98 | 39.2 |
| 100% MeOH eluate | 0.32 | 54.4 |

**Example 3.** This example illustrates the isolation of a high theobromine content solid preparation from the theobromine-enriched faction. The theobromine-enriched faction was obtained as in Example 1. The water rinse was collected and reduced to a low volume (to the point where cloudiness became apparent). A small amount of active carbon was added and the mixture filtered. Hot water was passed through the residue and the washings collected and added to the filtrate. The solution was reduced to a volume of 100 ml and poured into a separating funnel. Beaker washings and methylene chloride (50 ml) were added, the mixture shaken (3 to 4 times), allowed to settle, and the lower phase (chlorinated solvent) ran out. Methylene chloride addition, the shaking, settling, and separation steps were repeated three times. The methylene chloride was then removed by drying over sodium sulfate, the latter removed by filtration, and the theobromine enriched product obtained by evaporation on a rotary evaporator. The theobromine content of the solid preparation, as determined by HPLC, was about 84 percent. The preparation of a similar solid composition prepared using chloroform rather than methylene chloride as solvent contained about 64 percent theobromine.

## Claims

1. A process for the manufacture of a theobromine-enriched composition and a polyphenol-enriched composition from cocoa shells, said process comprising
(1) providing defatted cocoa shells;
(2) extracting the defatted cocoa shells with an aqueous acetone solution containing about 30 to about 80 percent acetone to provide a liquid cocoa shell extract material;
(3) treating the cocoa shell extract material to remove suspended solids to provide a treated liquid cocoa shell extract material;
(4) removing acetone from the treated liquid shell extract material to provide an aqueous extract material;
(5) concentrating the aqueous extract material to about 1.5 to about 5 percent solids to provide a concentrated extract material;
(6) applying the concentrated extract material to a gel filtration column containing a gel filtration medium suitable for separating theobromine from polyphenols;
(7) rinsing the gel filtration column with water to collect a theobromine-enriched composition; and
(8) eluting the water-rinsed gel filtration column with a low molecular weight organic solvent to collect a polyphenol-enriched composition, wherein the polyphenol-enriched composition is essentially free of phytosterols and theobromine.

2. The method of claim 1, wherein the aqueous acetone solution contains about 40 to about 60 percent acetone.

3. The method of claim 1, wherein the aqueous acetone solution contains about 50 percent acetone.

4. The method of claim 2, wherein the theobromine-enriched composition is treated to concentrate theobromine in the theobromine-enriched composition.

5. The method of claim 2, wherein the polyphenol-enriched fraction is treated to concentrate polyphenols in the polyphenol-enriched composition.

6. The method of claim 5, wherein the polyphenol-enriched fraction is treated to concentrate polyphenols in the polyphenol-enriched composition.

7. The method of claim 1, wherein the extraction in step (2) is repeated 2 to 5 times and the liquid cocoa shell extract material from each repeated extraction is combined.

8. The method of claim 2, wherein the extraction in step (2) is repeated 2 to 5 times and the liquid cocoa shell extract material from each repeated extraction is combined.

9. The method of claim 1, wherein the cocoa shells are obtained from roasted fermented cocoa beans.

10. The method of claim 1, wherein the cocoa shells are obtained from roasted unfermented cocoa beans.

11. The method of claim 1, wherein the cocoa shells are obtained from unroasted fermented cocoa beans.

12. The method of claim 1, wherein the cocoa shells are obtained from unroasted unfermented cocoa beans.

## Patentansprüche

1. Verfahren zur Herstellung einer Theobromin-angereicherten Zusammensetzung und einer Polyphenol-angereicherten Zusammensetzung aus Kakaobohnenschalen, wobei das Verfahren die folgenden Schritte umfaßt:
(1) das Bereitstellen entfetteter Kakaobohnenschalen;
(2) das Extrahieren der entfetteten Kakaobohnenschalen mit einer wäßrigen Acetonlösung, die ungefähr 30 bis ungefähr 80 % Aceton enthält, um ein flüssiges Kakaobohnenschalen-Extraktmaterial bereitzustellen;
(3) das Behandeln des Kakaobohnenschalen-Extraktmaterials, um suspendierte Feststoffe zu entfernen, um ein behandeltes flüssiges Kakaobohnenschalen-Extraktmaterial bereitzustellen;
(4) das Entfernen von Aceton aus dem behandelten flüssigen Schalenextraktmaterial, um ein wäßrigen Extraktmaterial bereitzustellen;
(5) das Konzentrieren des wäßrigen Extraktmaterials auf ungefähr 1,5 bis ungefähr 5 % Feststoffe, um ein konzentriertes Extraktmaterial bereitzustellen;
(6) das Auftragen des konzentrierten Extraktmaterials auf eine Gelfiltrationssäule, die ein Gelfiltrationsmedium enthält, das zum Trennen von Theobromin von Polyphenolen geeignet ist;
(7) das Spülen der Gelfiltrationssäule mit Wasser, um eine Theobromin-angereicherte Zusammensetzung zu erhalten; und
(8) das Eluieren der wassergespülten Gelfiltrationssäule mit einem niedermolekularen organischen Lösungsmittel, um eine Polyphenol-angereicherte Zusammensetzung zu erhalten, worin die Polyphenol-angereicherte Zusammensetzung im wesentlichen frei von Phytosterolen und Theobromin ist.

2. Verfahren gemäß Anspruch 1, worin die wäßrige Acetonlösung ungefähr 40 bis ungefähr 60 % Aceton enthält.

3. Verfahren gemäß Anspruch 1, worin die wäßrige Acetonlösung ungefähr 50 % Aceton enthält.

4. Verfahren gemäß Anspruch 2, worin die Theobromin-angereicherte Zusammensetzung behandelt wird, um Theobromin in der Theobromin-angereicherten Zusammensetzung anzureichern.

5. Verfahren gemäß Anspruch 2, worin die Polyphenol-angereicherte Fraktion behandelt wird, um Polyphenole in der Polyphenol-angereicherten Zusammensetzung anzureichern.

6. Verfahren gemäß Anspruch 5, worin die Polyphenol-angereicherte Fraktion behandelt wird, um Polyphenole in der Polyphenol-angereicherten Zusammensetzung anzureichern.

7. Verfahren gemäß Anspruch 1, worin die Extraktion in Schritt (2) 2- bis 5-mal wiederholt wird und das flüssige Kakaobohnenschalen-Extraktmaterial aus jeder wiederholten Extraktion kombiniert wird.

8. Verfahren gemäß Anspruch 2, worin die Extraktion in Schritt (2) 2- bis 5-mal wiederholt wird und das flüssige Kakaobohnenschalen-Extraktmaterial aus jeder wiederholten Extraktion kombiniert wird.

9. Verfahren gemäß Anspruch 1, worin die Kakaobohnenschalen von gerösteten fermentierten Kakaobohnen erhalten werden.

10. Verfahren gemäß Anspruch 1, worin die Kakaobohnenschalen von gerösteten unfermentierten Kakaobohnen erhalten werden.

11. Verfahren gemäß Anspruch 1, worin die Kakaobohnenschalen von ungerösteten fermentierten Kakaobohnen erhalten werden.

12. Verfahren gemäß Anspruch 1, worin die Kakaobohnenschalen von ungerösteten unfermentierten Kakaobohnen erhalten werden.

## Revendications

1. Procédé pour la production d'une composition enrichie en théobromine et d'une composition enrichie en polyphénols à partir de coques de cacao, ledit procédé comprenant les étapes consistant :
(1) à fournir des coques de cacao dégraissées ;
(2) à soumettre à une extraction les coques de cacao dégraissées avec une solution aqueuse d'acétone contenant environ 30 à environ 80 pourcent d'acétone pour fournir une matière consistant en un extrait liquide de coques de cacao ;
(3) à traiter la matière consistant en extrait de coques de cacao pour éliminer les matières solides en suspension afin de fournir une matière consistant en un extrait liquide traité de coques de cacao ;
(4) à éliminer l'acétone de la matière consistant en extrait liquide traité de coques pour fournir une matière consistant en un extrait aqueux ;
(5) à concentrer la matière consistant en extrait aqueux à une teneur en matières solides d'environ 1,5 à environ 5 pourcent pour fournir une matière consistant en un extrait concentré ;
(6) à appliquer la matière consistant en extrait concentré à une colonne de filtration sur gel contenant un milieu de filtration sur gel convenable pour séparer la théobromine des polyphénols ;
(7) à rincer la colonne de filtration sur gel avec de l'eau pour recueillir une composition enrichie en théobromine ; et
(8) à éluer la colonne de filtration sur gel rincée à l'eau avec un solvant organique de bas poids moléculaire pour recueillir une composition enrichie en polyphénols, ladite composition enrichie en polyphénols étant pratiquement dépourvue de phytostérols et de théobromine.

2. Procédé suivant la revendication 1, dans lequel la solution aqueuse d'acétone contient environ 40 à environ 60 pourcent d'acétone.

3. Procédé suivant la revendication 1, dans lequel la solution aqueuse d'acétone contient environ 50 d'acétone.

4. Procédé suivant la revendication 2, dans lequel la composition enrichie en théobromine est traitée pour concentrer la théobromine dans la composition enrichie en théobromine.

5. Procédé suivant la revendication 2, dans lequel la fraction enrichie en polyphénols est traitée pour concentrer les polyphénols dans la composition enrichie en polyphénols.

6. Procédé suivant la revendication 5, dans lequel la fraction enrichie en polyphénols est traitée pour concentrer les polyphénols dans la composition enrichie en polyphénols.

7. Procédé suivant la revendication 1, dans lequel l'extraction dans l'étape (2) est répétée 2 à 5 fois et la matière consistant en extrait liquide de coques de cacao de chaque extraction répétée est combinée.

8. Procédé suivant la revendication 2, dans lequel l'extraction dans l'étape (2) est répétée 2 à 5 fois et la matière consistant en extrait liquide de coques de cacao de chaque extraction répétée est combinée.

9. Procédé suivant la revendication 1, dans lequel les coques de cacao sont obtenues à partir de fèves de cacao fermentées torréfiées.

10. Procédé suivant la revendication 1, dans lequel les coques de cacao sont obtenues à partir de fèves de cacao non fermentées torréfiées.

11. Procédé suivant la revendication 1, dans lequel les coques de cacao sont obtenues à partir de fèves de cacao fermentées non torréfiées.

12. Procédé suivant la revendication 1, dans lequel les coques de cacao sont obtenues à partir de fèves de cacao non fermentées non torréfiées.
